# EUROPEAN PATENT SPECIFICATION

(11) **EP 4 193 946 B1**
(45) Date of publication and mention of the grant of the patent: **21.05.2025**
(21) Application number: 22212459.6
(22) Date of filing: 09.12.2022
(51) Int. Cl.: A61B 18/14, A61B 5/367, A61B 34/20, A61B 5/06, A61B 5/36, A61B 17/00, A61B 5/00, A61B 18/00, A61B 90/00

(54) **ELECTRICAL PATHS ALONG FLEXIBLE SECTION OF DEFLECTABLE SHEATH**
ELEKTRISCHE LEITUNGEN ENTLANG EINES FLEXIBLEN ABSCHNITTS EINER ABLENKBAREN HÜLLE
CHEMINS ÉLECTRIQUES LE LONG D'UNE SECTION FLEXIBLE D'UNE GAINE DÉFLECTABLE

(30) Priority: 10.12.2021 US 202117547517
(43) Date of publication of application: 14.06.2023
(73) Proprietor: Biosense Webster (Israel) Ltd, 2066717 Yokneam (IL)
(72) Inventor: SUAREZ, Paul A., Irvine 92618 (US); HIGHSMITH, Debby E., Irvine 92618 (US); BAR-TAL, Meir, Yokneam 2066717 (IL); LOVEJOY, Erica E., Irvine 92618 (US); HAIMOVICH, Roee, Yokneam 2066717 (IL); YAKOBOVITCH, Ohad, Yokneam 2066717 (IL)
(74) Representative: Carpmaels & Ransford LLP

(56) References cited:
- US-A1- 2007 016 007
- US-A1- 2018 110 562
- US-A1- 2021 077 180

## Description

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by application of energy (e.g., radiofrequency (RF) energy), it may be possible to cease or modify the propagation of unwanted electrical signals from one portion of the heart to another. The ablation process may provide a barrier to unwanted electrical pathways by creating electrically insulative lesions or scar tissue that effectively block communication of aberrant electrical signals across the tissue.

In some procedures, a catheter with one or more RF electrodes may be used to provide ablation within the cardiovascular system. The catheter may be inserted into a major vein or artery (e.g., the femoral artery) and then advanced to position the electrodes within the heart or in a cardiovascular structure adjacent to the heart (e.g., the pulmonary vein). The one or more electrodes may be placed in contact with cardiac tissue or other vascular tissue and then activated with RF energy to thereby ablate the contacted tissue. In some cases, the electrodes may be bipolar. In some other cases, a monopolar electrode may be used in conjunction with a ground pad or other reference electrode that is in contact with the patient that is in contact with the patient. Irrigation may be used to draw heat from ablating components of an ablation catheter; and to prevent the formation of blood clots near the ablation site.

An example of an ablation catheter is provided in US 2018/110562 A1. Further examples of ablation catheters are described in U.S. Pat. No. 10,743,932, entitled "Integrated Ablation System using Catheter with Multiple Irrigation Lumens," issued August 18, 2020; U.S. Pat. No. 10,660,700, entitled "Irrigated Balloon Catheter with Flexible Circuit Electrode Assembly," issued May 26, 2020; U.S. Pub. No. 2018/0071017, entitled "Ablation Catheter with a Flexible Printed Circuit Board," published March 15, 2018 ; U.S. Pat. No. 10,702,177, entitled "Catheter with Bipole Electrode Spacer and Related Methods," issued July 7, 2020; U.S. Pat. No. 10,130,422, entitled "Catheter with Soft Distal Tip for Mapping and Ablating Tubular Region," issued November 20, 2018; U.S. Pat. No. 8,956,353, entitled "Electrode Irrigation Using Micro-Jets," issued February 17, 2015; and U.S. Pat. No. 9,801,585, entitled "Electrocardiogram Noise Reduction," issued October 31, 2017.

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998. Examples of EP mapping catheters are described in U.S. Pat. No. 9,907,480, entitled "Catheter Spine Assembly with Closely-Spaced Bipole Microelectrodes," issued March 6, 2018; U.S. Pat. No. 10,130,422, entitled "Catheter with Soft Distal Tip for Mapping and Ablating Tubular Region," issued November 20, 2018; and U.S. Pat. No. 10,702,177, entitled "Catheter with Bipole Electrode Spacer and Related Methods," issued July 7, 2020.

When using an ablation catheter, it may be desirable to ensure that the one or more electrodes of the ablation catheter are sufficiently contacting target tissue. For instance, it may be desirable to ensure that the one or more electrodes are contacting target tissue with enough force to effectively apply RF ablation energy to the tissue; while not applying a degree of force that might tend to undesirably damage the tissue. To that end, it may be desirable to include one or more force sensors or pressure sensors to detect sufficient contact between one or more electrodes of an ablation catheter and target tissue.

In addition to using force sensing or EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued November 1, 2016; and various other references that are cited herein.

While several catheter systems and methods have been made and used, it is believed that no one prior to the inventors has made or used the invention described, illustrated and claimed herein.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient;
FIG. 2 depicts a perspective view of the catheter assembly of FIG. 1;
FIG. 3 depicts a perspective view of an end effector of the catheter assembly of FIG. 1;
FIG. 4 depicts a perspective view of an example of a guiding sheath that may be used with the catheter assembly of FIG. 1;
FIG. 5 depicts a distal portion of an alternative shaft assembly that may be integrated into the guiding sheath of FIG. 4;
FIG. 6 depicts a top plan view of a distal flex circuit of the shaft assembly of FIG. 5, with the distal flex circuit in a flat configuration;
FIG. 7 depicts a bottom plan view of the distal flex circuit of FIG. 6, with the distal flex circuit in the flat configuration;
FIG. 8 depicts another perspective view of the shaft assembly of FIG. 5, showing a wire lumen formed along a flexible region of the shaft assembly;
FIG. 9 depicts an enlarged top plan view of the distal flex circuit of FIG. 6, showing wires extending from the distal flex circuit into the wire lumen of FIG. 8;
FIG. 10 depicts an enlarged top plan view of a proximal flex circuit of the shaft assembly of FIG. 5, showing wires extending from the wire lumen of FIG. 8 to the proximal flex circuit;
FIG. 11A depicts an enlarged view of the wire lumen of FIG. 8, with one of the wires of FIGS. 9-10 in a bunched configuration;
FIG. 11B depicts an enlarged view of the wire lumen of FIG. 8, with one of the wires of FIGS. 9-10 in an elongated configuration;
FIG. 12 depicts a flow chart showing an example of a process that may be used to form a portion of the shaft assembly of FIG. 5;
FIG. 13 depicts a cross-sectional view showing a plastic material passing through an opening formed through one of the flex circuits of the shaft assembly of FIG. 5, with the plastic material forming a rivet configuration, as formed through the process of FIG. 12;
FIG. 14 depicts a flow chart showing an example of another process that may be used to form a portion of the shaft assembly of FIG. 5;
FIG. 15 depicts a cross-sectional view showing a plastic material passing through an opening formed through one of the flex circuits of the shaft assembly of FIG. 5, with the plastic material forming a rivet configuration, as formed through the process of FIG. 14; and
FIG. 16 depicts a schematic view of the end effector of FIG. 3 projecting distally from the shaft assembly of FIG. 5, with the shaft assembly in a deflected state.

### DETAILED DESCRIPTION FOR MODES OF CARRYING OUT THE INVENTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. The invention is defined by the scope of the appended claims. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 70% to 110%. In addition, as used herein, the terms "patient," "host," "user," and "subject" refer to any human or animal subject and are not intended to limit the systems or methods to human use, although use of the subject invention in a human patient represents a preferred embodiment.

### I. Overview of Example of a Catheter System

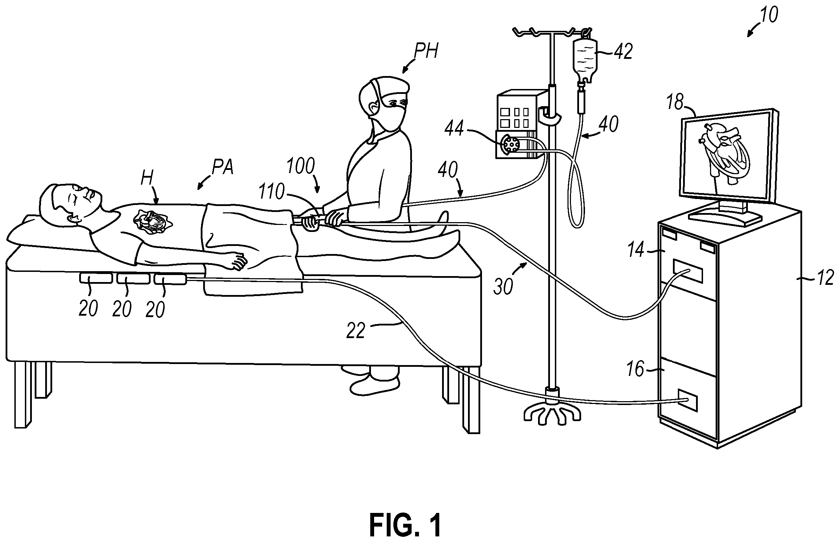

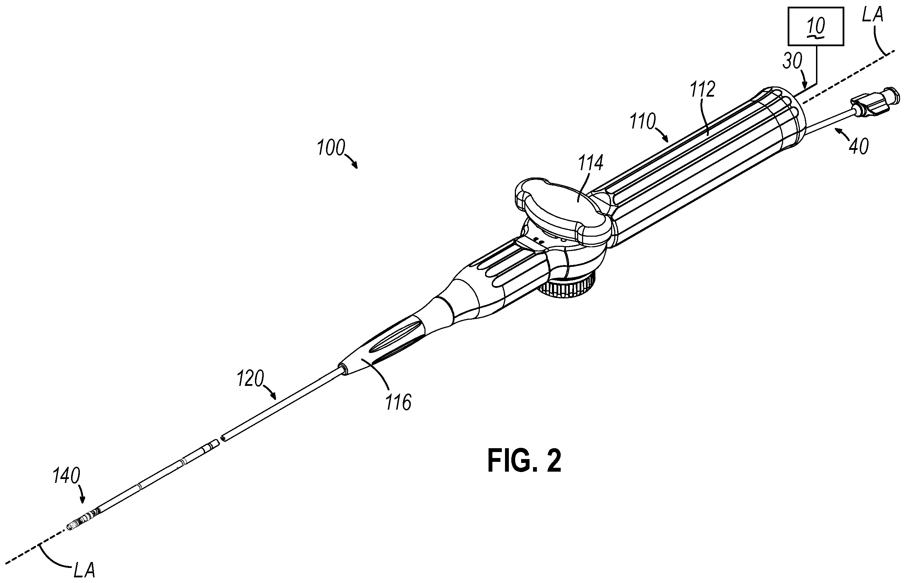

FIG. 1 shows an exemplary medical procedure and associated components of a cardiac catheter system that may be used to provide EP mapping or cardiac ablation as referred to above. In particular, FIG. 1 shows a physician (PH) grasping a handle assembly (110) of a catheter assembly (100), with an end effector (140) of a catheter (120) (shown in FIGS. 2-3 but not shown in FIG. 1) of catheter assembly (100) disposed in a patient (PA) to map potentials in tissue or ablate tissue in or near the heart (H) of the patient (PA). As shown in FIG. 2, catheter assembly (100) includes handle assembly (110), catheter (120) extending distally from handle assembly (110), end effector (140) located at a distal end of catheter (120), and a deflection drive actuator (114) associated with handle assembly (110).

As will be described in greater detail below, end effector (140) includes various components configured to deliver RF energy to targeted tissue sites, provide EP mapping functionality, track external forces imparted on end effector (140), track the location of end effector (140), or disperse irrigation fluid. Deflection drive actuator (114) is rotatable relative to a casing (112) of handle assembly (110) to thereby deflect end effector (140) and a distal portion of catheter (120) away from a central longitudinal axis (LA) defined by a proximal portion of catheter (120). Various suitable components that may be coupled with deflection drive actuator (114) and catheter (120) to provide such functionality will be apparent to those skilled in the art in view of the teachings herein.

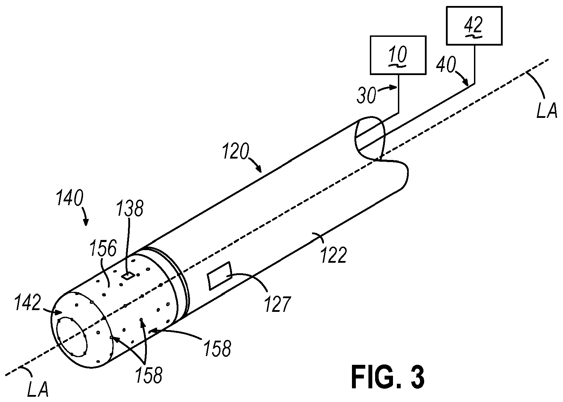

As shown in FIG. 3, catheter (120) includes an elongate flexible shaft (122), with end effector (140) extending distally from shaft (122). The proximal end of catheter (120) extends distally from a nozzle member (116) of handle assembly (110). In some versions, a heat shrink wrap (not shown) is provided about catheter (120), at the junction of the proximal end of catheter (120) and nozzle member (116). End effector (140) at the distal end of catheter (120) will be described in greater detail below. Catheter assembly (100) is coupled with a guidance and drive system (10) via a cable (30). Catheter assembly (100) is also coupled with a fluid source (42) via a fluid conduit (40). A set of field generators (20) are positioned underneath the patient (PA) and are coupled with guidance and drive system (10) via another cable (22). Field generators (20) are merely optional.

Guidance and drive system (10) of the present example include a console (12) and a display (18). Console (12) includes a first driver module (14) and a second driver module (16). First driver module (14) is coupled with catheter assembly (100) via cable (30). In some variations, first driver module (14) is operable to receive EP mapping signals obtained via microelectrodes (138) of end effector (140) as described in greater detail below. Console (12) includes a processor (not shown) that processes such EP mapping signals and thereby provides EP mapping as is known in the art.

First driver module (14) of the present example is further operable to provide RF power to a distal tip member (142) of end effector (140), as will be described in greater detail below, to thereby ablate tissue. Second driver module (16) is coupled with field generators (20) via cable (22). Second driver module (16) is operable to activate field generators (20) to generate an alternating magnetic field around the heart (H) of the patient (PA). For instance, field generators (20) may include coils that generate alternating magnetic fields in a predetermined working volume that contains the heart (H).

First driver module (14) is also operable to receive position indicative signals from a navigation sensor assembly (127) in catheter (120) near end effector (140). In such versions, the processor of console (12) is also operable to process the position indicative signals from navigation sensor assembly (127) to thereby determine the position of end effector (140) within the patient (PA). In some versions, navigation sensor assembly (127) includes two or more coils that are operable to generate signals that are indicative of the position and orientation of end effector (140) within the patient (PA). The coils are configured to generate electrical signals in response to the presence of an alternating electromagnetic field generated by field generators (20). Other components and techniques that may be used to generate real-time position data associated with end effector (140) may include wireless triangulation, acoustic tracking, optical tracking, inertial tracking, and the like. While navigation sensor assembly (127) is shown as being disposed in the distal end of catheter (120), navigation sensor assembly (127) may instead be positioned in end effector (140). Alternatively, catheter (120) and end effector (140) may lack a navigation sensor assembly (127).

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from navigation sensor assembly (127) of end effector (140). For instance, as end effector (140) of catheter (120) moves within the patient (PA), the corresponding position data from navigation sensor assembly (127) may cause the processor of console (12) to update the patient anatomy views in display (18) in real time to depict the regions of patient anatomy around end effector (140) as end effector (140) moves within the patient (PA). Moreover, the processor of console (12) may drive display (18) to show locations of aberrant conductive tissue sites, as detected via electrophysiological (EP) mapping with end effector (140) or as otherwise detected (e.g., using a dedicated EP mapping catheter, etc.). By way of example only, the processor of console (12) may drive display (18) to superimpose the locations of aberrant conductive tissue sites on the images of the patient's anatomy, such as by superimposing an illuminated dot, a crosshair, or some other form of visual indication of aberrant conductive tissue sites.

The processor of console (12) may also drive display (18) to superimpose the current location of end effector (140) on the images of the patient's anatomy, such as by superimposing an illuminated dot, a crosshair, a graphical representation of end effector (140), or some other form of visual indication. Such a superimposed visual indication may also move within the images of the patient anatomy on display (18) in real time as the physician moves end effector (140) within the patient (PA), thereby providing real-time visual feedback to the operator about the position of end effector (140) within the patient (PA) as end effector (140) moves within the patient (PA). The images provided through display (18) may thus effectively provide a video tracking the position of end effector (140) within a patient (PA), without necessarily having any optical instrumentation (i.e., cameras) viewing end effector (140). In the same view, display (18) may simultaneously visually indicate the locations of aberrant conductive tissue sites detected through EP mapping. The physician (PH) may thus view display (18) to observe the real time positioning of end effector (140) in relation to the mapped aberrant conductive tissue sites and in relation to images of the adjacent anatomical structures in the patient (PA).

Fluid source (42) of the present example includes a bag containing saline or some other suitable irrigation fluid. Conduit (40) includes a flexible tube that is further coupled with a pump (44), which is operable to selectively drive fluid from fluid source (42) to catheter assembly (100). As described in greater detail below, such irrigation fluid may be expelled through openings (158) of distal tip member (142) of end effector (140). Such irrigation may be provided in any suitable fashion as will be apparent to those skilled in the art in view of the teachings herein.

### II. Example of an End Effector

As mentioned above, end effector (140) includes various components configured to deliver RF energy to targeted tissue sites, provide EP mapping functionality, track external forces imparted on end effector (140), track the location of end effector (140) within the patient (PA), and emit irrigation fluid. As shown in FIG. 3, end effector (140) of the present example includes a distal tip member (142), which further includes a cylindraceous body (156) with a dome tip. Cylindraceous body (156) and the dome tip may be formed of an electrically conductive material, such as metal. A plurality of openings (158) are formed through cylindraceous body (156) and are in communication with the hollow interior of distal tip member (142). Openings (158) thus allow irrigation fluid to be communicated from the interior of distal tip member (142) out through cylindraceous body (156). Cylindraceous body (156) and the dome tip are also operable to apply RF electrical energy to tissue to thereby ablate the tissue. Such RF electrical energy may be communicated from first driver module (14). Distal tip member (142) may also include one or more thermocouples that are configured to provide temperature sensing capabilities. This may prevent overheating of distal tip member (142) or adjacent tissue.

As also shown in FIG. 3, distal tip member (142) of the present example also includes one or more EP mapping microelectrodes (138) mounted to cylindraceous body (156). EP mapping microelectrodes (138) are configured to pick up electrical potentials from tissue that comes into contact with EP mapping microelectrodes (138). EP mapping microelectrodes (138) may thus be used to determine locations of aberrant electrical activity in tissue within a cardiovascular anatomical structure (e.g., pulmonary vein, etc.). Signals picked up by EP mapping microelectrodes (138) may be communicated to first driver module (14) of console (12) via cable (30). First driver module (14) may process the EP mapping signals and provide the physician (PH) with corresponding feedback indicating the locations of aberrant electrical activity in accordance with the teachings of various references cited herein.

In versions where cylindraceous body (156) is formed of an electrically conductive material to provide RF electrical energy for tissue ablation, an electrically insulating material may be interposed between cylindraceous body (156) and EP mapping microelectrodes (138) to thereby electrically isolate EP mapping microelectrodes (138) from cylindraceous body (156). EP mapping microelectrodes (138) may be constructed and operable in accordance with the teachings of various patent references cited herein. While only one EP mapping microelectrode (138) is shown, distal tip member (142) may include two or more EP mapping microelectrodes (138). Alternatively, distal tip member (142) may lack EP mapping microelectrodes (138) altogether.

In some variations, end effector (140) may further include force sensor that is configured to sense external forces that impinge against distal tip member (142). By way of example only, such a force sensor may take the form of a strain gauge or any other suitable component(s). When distal tip (142) encounters external forces (e.g., when distal tip (142) is pressed against tissue), those external forces are communicated from distal tip (142) to the force sensor, such that the force sensor may generate a suitable signal corresponding to the magnitude and direction of the external force. The signals from the force sensor may be communicated to first driver module (14) of console (12) via cable (30). First driver module (14) may process the strain signals in accordance with any suitable fashion as would be apparent to one skilled in the art in view of the teachings herein. By way of example only, console (12) may provide audible feedback to alert the physician (PH) when the force sensor indicates that distal tip member (142) is encountering forces over a predetermined threshold, to thereby prevent the physician (PH) from unwittingly damaging a cardiovascular anatomical structure with distal tip member (142). In some versions, the force sensor may be omitted.

In addition to the foregoing, end effector (140) and other aspects of catheter assembly (100) may be configured and operable in accordance with at least some of the teachings of any one or more of the various patent documents that are discussed in the present disclosure. Alternatively, end effector (140) may have any other suitable components, features, and capabilities.

### III. Example of Guiding Sheath

In some procedures, the physician (PH) may wish to introduce catheter (120) into the patient (PA) via a guiding sheath (200). In some such procedures, the guiding sheath may be inserted into the patient (PA) (e.g., via the leg or groin of the patient (PA)); and then be advanced along a vein or artery to reach a position in or near the heart (H). Once the guiding sheath is suitably positioned in the patient (PA), the physician (PA) may then advance end effector (140) and catheter (120) into the guiding sheath until end effector (140) exits the distal end of the guiding sheath. The physician (PA) may then operate catheter assembly (100) to provide EP mapping, ablation, or any other kind of operations in or near the heart (H) of the patient (PA).

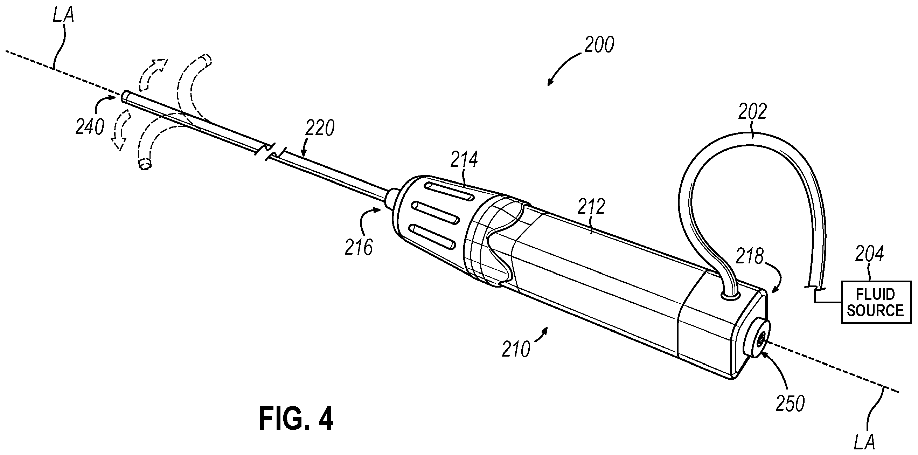

FIG. 4 shows an example of a guiding sheath (200) that may be used in such procedures. Guiding sheath (200) of this example includes a body in the form of a handle assembly (210) with a hollow shaft (220) extending distally from a distal end (216) of handle assembly (210). Handle assembly (210) is configured for grasping by a casing (212). The open distal end (240) of the hollow shaft (220) is operable to deflect laterally away from a longitudinal axis (LA) of the shaft. This deflection is controlled by a rotary knob (214) at distal end (216) of handle assembly (210). Rotary knob (214) is rotatable relative to casing (212), about the longitudinal axis (LA), to thereby actuate components that drive lateral deflection of open distal end (240) of hollow shaft (220). By way of example only, such actuation components may include one or more pull wires, bands, or any other suitable structures as will be apparent to those skilled in the art in view of the teachings herein. While the body of guiding sheath (200) is in the form of handle assembly (210) in this example, the body may take other forms. For instance, some variations may provide a robotically controlled form of guiding sheath (200). In some such variations, the body may take the form of a structure that mechanically interfaces with a robotic arm and/or other kind of robotic driving feature.

As shown in FIG. 4, a tube (202) extends laterally from the proximal end (218) of handle assembly (210). Tube (202) of this example is in fluid communication with a hollow interior (not shown) defined within handle assembly (210), with the hollow interior being in fluid communication with the interior of hollow shaft (220). Tube (202) of the present example is further in fluid communication with a fluid source (204). By way of example only, fluid source (204) may contain saline or any other suitable fluid. In some instances, fluid from fluid source (204) is communicated through tube (202), a hollow interior region defined within handle assembly (210), and the interior of hollow shaft (220), to thereby flush the fluid path defined by tube (202), the hollow interior region defined within handle assembly (210), and the interior of hollow shaft (220).

As also shown in FIG. 4, proximal end (218) of handle assembly (210) further includes an insertion port (250). Insertion port (250) is aligned with the longitudinal axis (LA) and provides a port for inserting end effector (140) and catheter (120) into hollow shaft (220) as will be described in greater detail below. Insertion port(250) of this example includes an annular protrusion (252) defining an opening (254). Protrusion (252) protrudes proximally from casing (212) at proximal end (218). In some versions, protrusion (252) is omitted.

A seal (260) is positioned within opening (254). By way of example only, seal (260) may include an elastomeric membrane or other kind of component(s) as will be apparent to those skilled in the art in view of the teachings herein. Seal (260) of the present example further includes a slit arrangement (262) that is configured to facilitate insertion of an instrument (e.g., catheter (120), etc.) through seal (260). In the present example, slit arrangement (262) is in the form of a "+" sign, though any other suitable kind of configuration may be used. When nothing is inserted through seal (260), seal (260) is configured to provide a fluid-tight seal that prevents fluid from escaping the portion of the above-described fluid path defined within handle assembly (210) via insertion port (250); and prevents air from entering the above-described fluid path defined within handle assembly (210) via insertion port (250). When an instrument is inserted through seal (260), seal (260) still substantially maintains a fluid-tight seal of port (250), preventing fluid from escaping the above-described fluid path defined within handle assembly (210) via insertion port (250); and preventing air from entering above-described fluid path defined within handle assembly (210) via insertion port (250), while still allowing the inserted instrument to translate relative to seal (260). Thus, regardless of whether an instrument is disposed in insertion port (250), seal (260) may prevent fluids from leaking out through insertion port (250) and prevent air from being aspirated into the heart (H) of the patient (PA) via insertion port (250).

### IV. Example of Alternative Sheath Shaft Assembly with Integral Circuits

As described above, a guiding sheath (200) may be useful in assisting a physician (PH) to guide another instrument, such as catheter assembly (100), to a target location within a patient (PA) (e.g., within the cardiovascular system of the patient (PA)). Such guidance may be facilitated by use of rotary knob (214) to drive lateral deflection of open distal end (240) of hollow shaft (220). As also described above, a navigation sensor assembly (127) in catheter (120) may be useful in assisting a physician (PH) to determine the position of end effector (140) within the patient (PA). As end effector (140) of catheter (120) moves within the patient (PA), the corresponding position data from navigation sensor assembly (127) may cause the processor of console (12) of guidance and drive system (10) to update the patient anatomy views in display (18) in real time, to depict a representation of end effector (140) in the regions of patient anatomy around end effector (140) as end effector (140) moves within the patient (PA).

In some scenarios, it may be further beneficial to provide a physician (PH) with an indication of where guiding sheath (200) is positioned in the patient (PA), in real time, via display (18). To that end, it may be desirable to incorporate one or more navigation sensor assemblies (e.g., like navigation sensor assembly (127)) in guiding sheath (200). An example of how this may be achieved will be discussed in greater detail below.

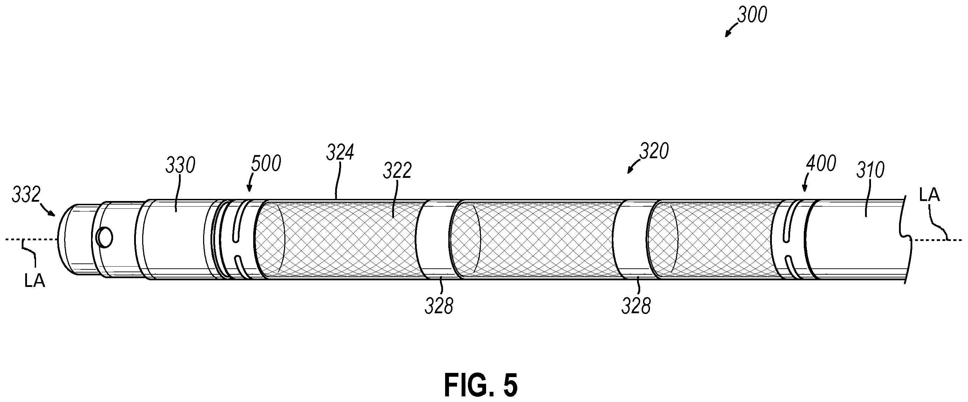

FIG. 5 depicts a distal portion of an example of an alternative shaft assembly (300) that may be incorporated into guiding sheath (200) in place of shaft (220) described above. Shaft assembly (300) of this example includes a proximal portion (310), a steerable portion (320) distal to proximal portion (310), and a distal portion (330) distal to steerable portion (320). Proximal portion (310) is laterally flexible and extends from handle assembly (210) to steerable portion (320). Steerable portion (320) includes an internal braided structure (322), a flexible outer layer (324), and rings (328) longitudinally spaced apart from each other. Distal portion (330) includes an open distal end (332). One or more wires, cables, bands, or other drive members are coupled with rotary knob (214) and are operable to drive lateral deflection of distal portion (330) at steerable portion (320). In some versions, such drive members are secured to distal portion (330). In some other versions, such drive members are secured to a distal region of steerable portion (330). In either arrangement, shaft assembly (300) is operable to bend laterally at steerable portion (320), in response to rotation of rotary knob (214), to thereby reorient distal portion (330). Any suitable components and arrangements may be used to drive such lateral deflection of distal portion (330) at steerable portion (320) in response to rotation of rotary knob (214). Moreover, any other suitable kind of actuator or actuators may be used in place of rotary knob (214).

Internal braided structure (322) of the present example may include a metallic material, a polymeric material, or any other suitable kind of material or combination of materials. Internal braided structure (322) may provide structural support to steerable portion (320), such as by preventing prolapse or buckling at steerable portion (320), while still allowing shaft assembly (300) to bend laterally at steerable portion (320). Flexible outer layer (324) may include any suitable kind of polymeric material or other kind of material(s) and may provide a substantially smooth outer surface along steerable portion (320).

Rings (328) of the present example are formed of a metallic or otherwise electrically conductive material. Rings (328) are exposed such that rings (328) may contact the blood of the patient (PA) while shaft assembly (300) is disposed in the patient (PA). Rings (328) may be used as reference electrodes to sense the impedance of the blood of the patient (PA). Impedance measurements from rings (328) may be used to allow for location sensing of the sheath member (320). The technique for location sensing via impedance measurement with ring electrodes is known in the art and is shown and described in US Patent Nos. 7,869,865; and 8,456,182. In some scenarios where rings (328) are used to provide impedance-based position sensing of shaft assembly (300), a plurality of additional electrodes (not shown) may be secured to the body of the patient (PA), such as an external skin surface. Electrical currents may be passed between rings (328) and the external electrodes. The respective characteristics of the currents passing between rings (328) and the external electrodes (e.g., impedance values) may be measured to determine the position coordinates of shaft assembly (300), as such values may vary based on the respective positioning between rings (328) and the external electrodes.

In addition to being used to provide location sensing as described above, in some versions, electrode rings (328) may be used to provide a reference signal during an EP mapping procedure, during an ablation procedure, or during any other kinds of procedures where blood impedance measurements may be useful. Such EP mapping procedures, ablation procedures, or other procedures may be performed via a catheter (e.g., catheter (120)) or some other instrument that is disposed in shaft assembly (300). In some scenarios, electrode rings (328) may contact tissue and may therefore pick up potentials from the tissue. While two rings (328) are shown, some variations may provide only one ring (328) or more than two rings (328). In some other variations, rings (328) are omitted from shaft assembly (300).

Shaft assembly (300) of the present example further includes a proximal flex circuit (400) and a distal flex circuit (500). Proximal flex circuit (400) is positioned at the distal end of proximal portion (310) and at the proximal end of steerable portion (320). In some versions, proximal flex circuit (400) is positioned entirely on proximal portion (310), proximal to steerable portion (320) but proximate to steerable portion (320). Distal flex circuit (500) is positioned at the proximal end of distal portion (330) and at the distal end of steerable portion (320). In some versions, distal flex circuit (500) is positioned entirely on distal portion (330), distal to steerable portion (320) but proximate to steerable portion (320).

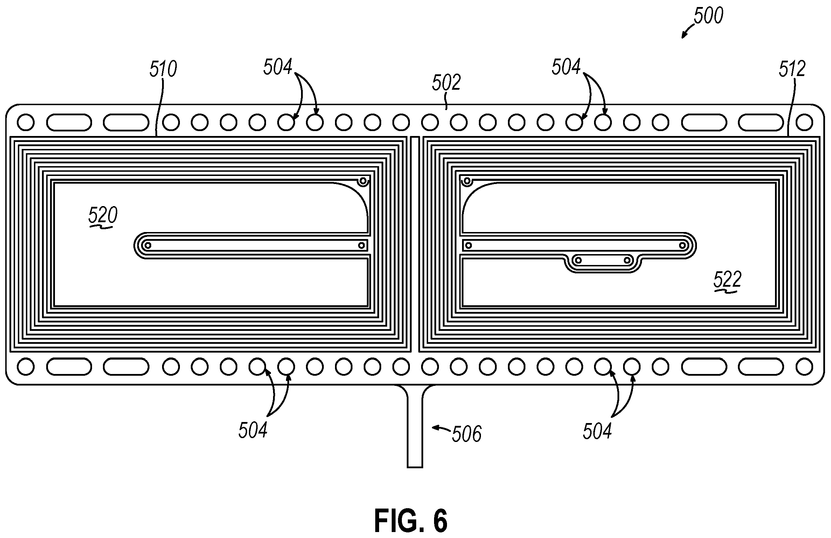

FIGS. 6-7 show various features of distal flex circuit (500) prior to the flex circuit being wrapped around the sheath (320). As shown in the unrolled form or planar form, distal flex circuit (500) of this example includes a flexible substrate (502) having a plurality of openings (504) formed therethrough. Flexible substrate (502) may be formed of polyimide material, liquid crystal polymer (LCP) material, and/or any other suitable kind(s) of material(s). Flexible substrate (502) also includes a tail portion (506) as will be described in greater detail below. While FIGS. 6-7 show distal flex circuit (500) in a flat configuration, it should be understood that distal flex circuit (500) is wrapped about distal portion (330) of shaft assembly (300), such that distal flex circuit (500) conforms to the curvature of distal portion (330) of shaft assembly (300), when shaft assembly (300) is fully assembled.

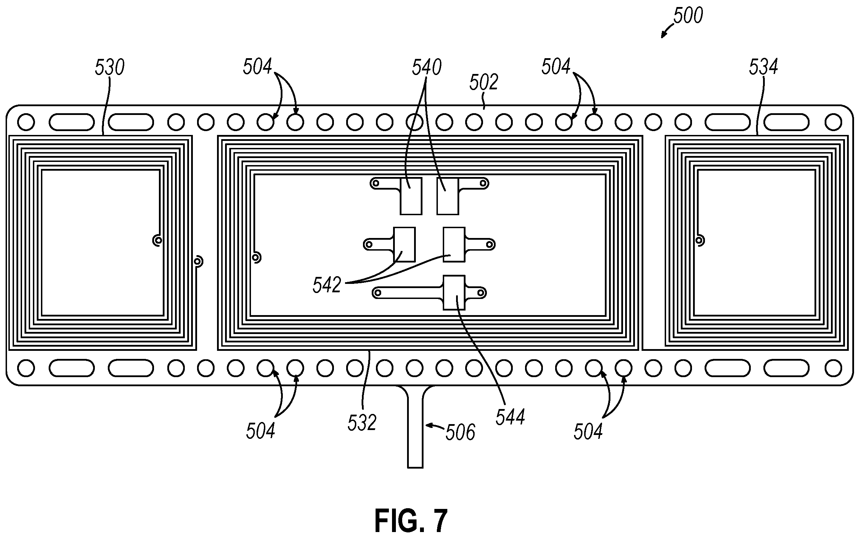

The face of distal flex circuit (500) shown in FIG. 6 faces outwardly from shaft assembly (300) when shaft assembly (300) is fully assembled; while the face of distal flex circuit (500) shown in FIG. 7 faces inwardly (i.e., toward the longitudinal axis (LA)) when shaft assembly (300) is fully assembled. The outer face of distal flex circuit (500) shown in FIG. 6 includes two coil portions (510, 512) that together form a first type of navigation sensor (e.g., an electromagnetic-based navigation sensor). When flex circuit (500) is wrapped about distal portion (330) of shaft assembly (300), coil portions (510, 512) are positioned diametrically opposite to each other about the outer diameter of distal portion (330). However, coil portions (510, 512) are electrically coupled together, such that coil portions (510, 512) cooperate to collectively form a single, larger navigation sensor coil. The navigation sensor formed by coil portions (510, 512) is configured to generate electrical signals in response to the presence of an alternating electromagnetic field generated by field generators (20) located outside the body, with such signals being indicative of the position and orientation of distal flex circuit (500) in the patient (PA) due to triangulation of the field generators (20).

In versions where rings (328) are used in combination with external electrodes to generate position data based on impedance values and/or other characteristics associated with current flowing between rings and the external electrodes, such impedance-based position data from rings (328) may be used in combination with the electromagnetic position data generated using the navigation sensor formed by coil portions (510, 512). For instance, in some scenarios the position data generated using the navigation sensor formed by coil portions (510, 512) may first be used to define an initial position matrix. Once that initial position matrix is defined based on the position data generated using the navigation sensor formed by coil portions (510, 512), the position data generated using rings (328) may be used to further refine the initial position matrix that is defined from the electromagnetic position data generated using the navigation sensor formed by coil portions (510, 512). Alternatively, the electromagnetic-based position sensing data may be used in combination with the impedance-based position sensing data in any other suitable fashion.

The outer face of distal flex circuit (500) shown in FIG. 6 further includes two electrode pads (520, 522). Electrode pads (520, 522) are configured to contact blood and measure impedance of the blood as described earlier in relation to location sensing by impedance or for measurement of EP mapping reference signals in blood via rings (328). In some such versions, electrode pads (520, 522) are used to obtain impedance measurements that are further used to provide location sensing as noted above with reference to US Patent Nos. 7,869,865; and 8,456,182. In other words, the electrode pads (520, 522) can be viewed of as a second type of navigation sensor, albeit one which in some cases might not be as accurate as the first type of navigation sensor (e.g., electromagnetic type location sensor) and therefore may require a location matrix to be generated before this second type of navigation sensor (e.g., impedance location sensing) can be viewed by the operator. In other words, impedance signals from electrode pads (520, 522) may be used in conjunction with signals from coil-based sensors (e.g., a navigation sensor formed by coil portions (510, 512)) to create a location matrix; similar to the teachings provided above in relation to rings (328). In some variations, electrode pads (520, 522) are used to contact tissue and thereby measure the impedance of the tissue for other purposes. While distal flex circuit (500) includes only two electrode pads (520, 522) in this example, distal flex circuit (500) may instead include just one electrode pad or more than two electrode pads. In some versions where distal flex circuit (500) includes electrode pads (520, 522), rings (328) may be omitted since electrode pads (520, 522) may perform functions similar to those performed by rings (328) as described above. Some versions may nevertheless include a combination of electrode pads (520, 522) and rings (328).

The inner face of distal flex circuit (500) shown in FIG. 7 includes three coil portions (530, 532, 534) that together form another navigation sensor that may operate like the above-described first type of navigation sensor, which utilizes the known magnetic triangulation technique with external magnetic field generators (20) underneath the patient's body. When flex circuit (500) is wrapped about distal portion (330) of shaft assembly (300), coil portions (530, 534) are positioned adjacent to each other and diametrically opposite to coil portion 532) about the outer diameter of distal portion (330). However, coil portions (530, 532, 534) are electrically coupled together, such that coil portions (530, 532, 534) cooperate to collectively form a single, larger navigation sensor coil. The navigation sensor formed by coil portions (530, 532, 534) is configured to generate electrical signals in response to the presence of an alternating electromagnetic field generated by field generators (20), with such signals being indicative of the position and orientation of distal flex circuit (500) in the patient (PA). As noted above with respect to the position data generated using the electromagnetic navigation sensor formed by coil portions (510, 512), the position data generated using the electromagnetic navigation sensor formed by coil portions (520, 532, 534) may be used in combination with the position data generated using the impedance-based navigation sensor formed by rings (328).

This single navigation sensor coil collectively formed by coil portions (530, 532, 534) is angularly offset from the single navigation sensor coil collectively formed by coil portions (510, 512) by about 90 degrees about the circumference of distal portion (330). Thus, the navigation sensor formed by coil portions (530, 532, 534) on the inner face of distal flex circuit (500) shown in FIG. 7 may provide one sensor axis while the navigation sensor formed by coil portions (510, 512) on the outer face of distal flex circuit (500) shown in FIG. 6 may provide another sensor axis, such that distal flex circuit (500) provides a double-axis sensor assembly. In some such configurations, the navigation sensor formed by coil portions (530, 532, 534) on the inner face of distal flex circuit (500) is independent of the navigation sensor formed by coil portions (510, 512) on the outer face of distal flex circuit (500). In some other variations, distal flex circuit (500) only provides a single-axis sensor assembly, provides a three-axis sensor assembly, or sensor assembly with any other suitable number of axes.

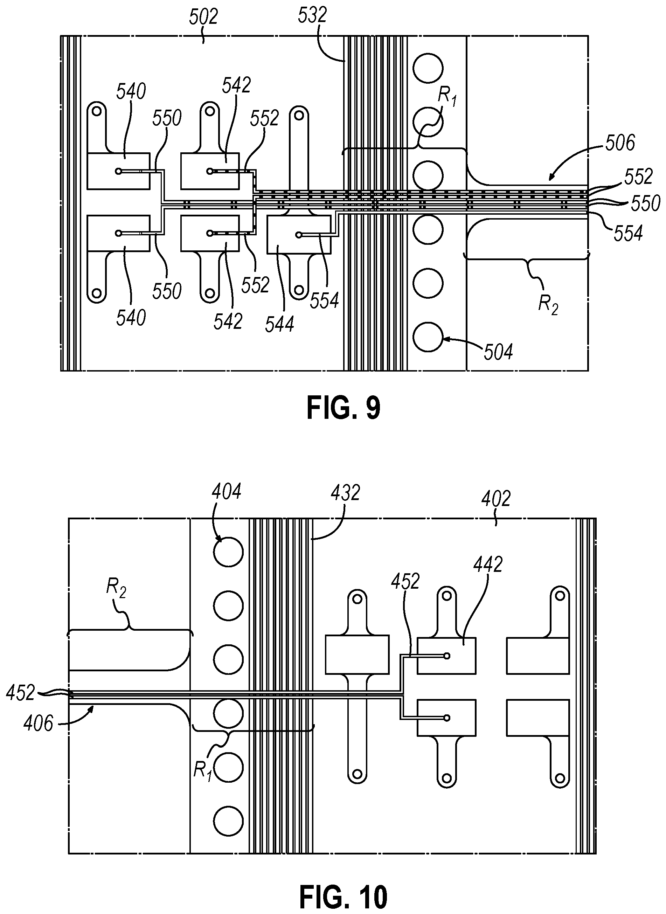

The inner face of distal flex circuit (500) shown in FIG. 7 further includes a set of connection pads (540, 542, 544). Connection pads (540, 542, 544) are configured to be coupled with corresponding wires (550, 552, 554) (e.g., via soldering, etc.), as shown in FIG. 9. Connection pads (540, 542, 544) serve as electrical connection points between wires (550, 552, 544) and electrical components of distal flex circuit (500). For instance, connection pads (540) may provide an electrical connection with coil portions (510, 512) on the outer face of distal flex circuit (500) shown in FIG. 6. Connection pads (542) may provide an electrical connection with coil portions (530, 532, 534) on the inner face of distal flex circuit (500) shown in FIG. 7. Connection pads (544) may provide an electrical connection with electrode pads (520, 522) on the outer face of distal flex circuit (500) shown in FIG. 6. Alternatively, any other suitable arrangements and relationships may be used. Electrical connections between connection pads (544) and the corresponding features of distal flex circuit (500) may be formed through traces in distal flex circuit (500). Any other suitable kinds of electrical connection features may be used in addition to, or in lieu of, connection pads (540, 542, 544).

Proximal flex circuit (400) may be configured and operable similar to distal flex circuit (500). Alternatively, proximal flex circuit (400) may lack certain features found in distal flex circuit (500) and/or include certain features that are not found in distal flex circuit (500). In the present example, and as shown in FIG. 10, proximal flex circuit (400) includes a flexible substrate (402) having a plurality of openings (404) formed therethrough. Flexible substrate (402) may be formed of polyimide material, liquid crystal polymer (LCP) material, and/or any other suitable kind(s) of material(s). Flexible substrate (402) also includes a tail portion (406). While FIG. 10 shows proximal flex circuit (400) in a flat configuration, it should be understood that proximal flex circuit (400) is wrapped about proximal portion (310) of shaft assembly (300), such that proximal flex circuit (400) conforms to the curvature of proximal portion (310) of shaft assembly (300), when shaft assembly (300) is fully assembled.

The face of proximal flex circuit (400) shown in FIG. 10 faces inwardly (i.e., toward the longitudinal axis (LA)) when shaft assembly (300) is fully assembled. This inner face of proximal flex circuit (400) includes at least one coil portion (432) that forms a navigation sensor. The navigation sensor formed by coil portion (432) (and perhaps other coil portions on the inner face of proximal flex circuit (400)) is configured to generate electrical signals in response to the presence of an alternating electromagnetic field generated by field generators (20), with such signals being indicative of the position and orientation of proximal flex circuit (400) in the patient (PA). In the present example, the outer face of proximal flex circuit (400) does not include coil portions that form a navigation sensor. However, in some variations, the outer face of proximal flex circuit (400) may include one or more coil portions that form a navigation sensor. Such a navigation sensor on the outer face of proximal flex circuit (400) may be provided in addition to, or in lieu of, the navigation sensor on the inner face of proximal flex circuit (400). Thus, proximal flex circuit (400) may provide a double-axis sensor assembly. Alternatively, proximal flex circuit (400) may provide a single-axis sensor assembly, provides a three-axis sensor assembly, or sensor assembly with any other suitable number of axes.

The inner face of proximal flex circuit (400) shown in FIG. 10 also includes a set of connection pads (442). Connection pads (442) are configured to be coupled with corresponding wires (452) (e.g., via soldering, etc.), as shown in FIG. 10. In the present example, connection pads (442) serve as electrical connection points between wires (452) and coil portion (432). Traces are formed through proximal flex circuit (400) to form electrical connections between connection pads (442) and coil portion (432). While proximal flex circuit (400) lacks other electrical components in the present example, other variations of proximal flex circuit (400) may include additional electrical components. For instance, some variations of proximal flex circuit (400) may include additional coil portions as noted above. In addition, or in the alternative, some variations of proximal flex circuit (400) may include electrode pads similar to electrode pads (520, 522) of distal flex circuit (500) as described above. In versions where proximal flex circuit (400) includes electrode pads, other distal flex circuit (500) may also include electrode pads (520, 522) or may lack electrode pads (520, 522).

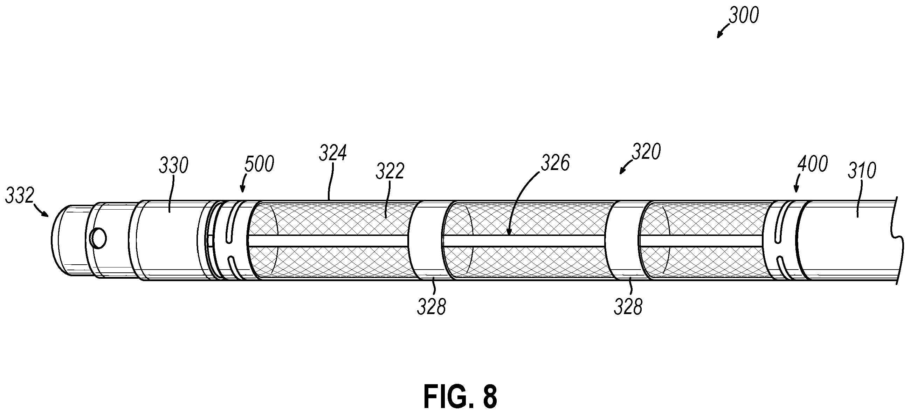

As shown in FIG. 8, steerable portion (320) of shaft assembly (300) includes a channel (326) that is configured to receive wires (452, 550, 552, 554). In some versions, channel (326) comprises a tube that is disposed in flexible outer layer (324). In some other versions, channel (326) comprises a lumen or void that is formed in an interior region of flexible outer layer (324), external to braided structure (322) but oriented such that wires (452, 550, 552, 554) in the lumen are not exposed to the environment outside of steerable portion (320). Alternatively, channel (326) may take any other suitable form. Moreover, a plurality of channels (326) may be provided in some versions. In some versions, channel (326) extends all the way proximally back to the proximal end of shaft assembly (300). In some other versions, channel (326) is in communication with another channel or lumen (not shown), and this other channel or lumen extends all the way proximally back to the proximal end of shaft assembly (300), such that wires (452, 550, 552, 554) initially enter channel (326) and then continue through the other channel or lumen to reach the proximal end of shaft assembly (300). In some such versions, this other lumen is formed in the wall of proximal portion (310). As yet another variation, wires (452, 550, 552, 554) (or a flex circuit including traces that effectively replace wires (452, 550, 552, 554)) is adhered to the exterior of proximal portion (310).

Regardless of how wires (452, 550, 552, 554) reach the proximal end of shaft assembly (300) wires (452, 550, 552, 554) may ultimately reach corresponding connection points in handle assembly (110). Such connection points in handle assembly (110) may provide an electrical interface between wires (452, 550, 552, 554) and cable (30), which may in turn provide a path for electrical communication with guidance and drive system (10).

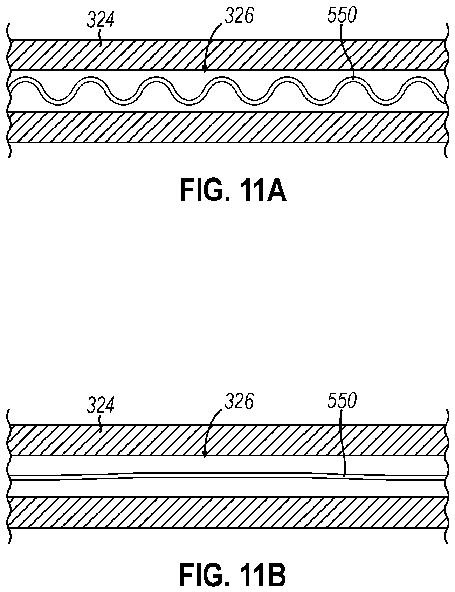

With wires (452, 550, 552, 554) extending along at least part of the length of steerable portion (320) via channel (326), it may be desirable to accommodate movement of wires (452, 550, 552, 554) relative to steerable portion (320) as steerable portion (320) is deflected laterally during operation of shaft assembly (300). In particular, when steerable portion (320) is deflected laterally during operation of shaft assembly (300), such lateral deflection may tend to change the effective length of the portions of wires (452, 550, 552, 554) disposed in channel (326). In the present example, to accommodate movement of wires (452, 550, 552, 554) relative to steerable portion (320), wires (452, 550, 552, 554) are provided in a bunched-up, serpentine configuration as shown in FIG. 11A when steerable portion (320) is in a straight configuration. While FIGS. 11A-11B only show wire (550), it should be understood that wires (452, 552, 554) may be configured similarly to wire (550) within channel (326). As shown in FIG. 11B, when steerable portion (320) is bent in a direction that would tend to increase the effective length of the region of wire (550) disposed in channel (326), the region of wire (550) disposed in channel (326) may substantially straighten. The bunched-up, serpentine configuration as shown in FIG. 11A may thus effectively function like a service loop, providing additional freedom of movement of wires (452, 550, 552, 554) within channel (326). In scenarios where deflection at steerable portion (320) causes a reduction in effective length, the service loop may provide predictable and controlled compression of wires (452, 550, 552, 554).

In some versions, wires (452, 550, 552, 554) are incorporated into an assembly that resiliently biases wires (452, 550, 552, 554) to achieve the bunched-up, serpentine configuration as shown in FIG. 11A within channel (326). Such an assembly may take the form of an elastic lamination, where wires (452, 550, 552, 554) are interposed between two sheets of stretchable plastic. By way of example only, the plastic material may include polyether block amide and/or any other suitable kind of material. By way of further example only, the plastic material may have a durometer of 25D or any other suitable durometer. The sheets of stretchable plastic and wires (452, 550, 552, 554) may be affixed together using any suitable features or techniques, including but not limited to pressure-sensitive adhesive, other adhesive, reflowing the plastic sheets together, etc. In use, the assembly of wires (452, 550, 552, 554) and the plastic sheets may provide wires (452, 550, 552, 554) in the bunched-up, serpentine configuration as shown in FIG. 11A within channel (326) when the assembly is not placed in tension (e.g., when steerable portion (320) is in a straight configuration). When the assembly is placed in tension (e.g., when steerable portion (320) is in a deflected configuration), the assembly may stretch to provide the substantially straightened configuration of wires (452, 550, 552, 554) shown in FIG. 11B. When that tension is removed (e.g., e.g., when steerable portion (320) returns to a straight configuration), the resilience of the plastic sheets may return wires (452, 550, 552, 554) to the bunched-up, serpentine configuration as shown in FIG. 11A.

It should be understood that wires (452, 550, 552, 554) may encounter tension or compression in response to the combination of the direction in which steerable portion (320) is bent and the side of steerable portion (320) on which wires (452, 550, 552, 554) are positioned (i.e., whether wires (452, 550, 552, 554) are on the inside of the curve or the outside of the curve). In some scenarios, depending on the combination of the direction in which steerable portion (320) is bent and the side of steerable portion (320) on which wires (452, 550, 552, 554) are positioned, wires (452, 550, 552, 554) may encounter neither tension nor compression.

As alternative to laminating wires (452, 550, 552, 554) between plastic sheets, each wire (452, 550, 552, 554) may be coated with a flexible material that resiliently biases each wire (452, 550, 552, 554) to assume the bunched-up, serpentine configuration as shown in FIG. 11A. As another variation, a single plastic sheet may be used instead of two sheets. For instance, wires (452, 550, 552, 554) may be placed on the single sheet of plastic, and then the plastic sheet may be folded over wires (452, 550, 552, 554) and then secured to wires (452, 550, 552, 554) to thereby bias wires (452, 550, 552, 554) to the bunched-up, serpentine configuration as shown in FIG. 11A. As another variation, wires (452, 550, 552, 554) may be placed on the single sheet of plastic, and then the plastic sheet may be rolled over wires (452, 550, 552, 554) and then secured to wires (452, 550, 552, 554) to thereby bias wires (452, 550, 552, 554) to the bunched-up, serpentine configuration as shown in FIG. 11A. As another variation, wires (452, 550, 552, 554) may be embedded within a single sheet of plastic and/or otherwise secured directly to the sheet of plastic, such that the sheet of plastic may thereby bias wires (452, 550, 552, 554) to the bunched-up, serpentine configuration as shown in FIG. 11A. Of course, one or more plastic sheets and/or other additional materials may be omitted, such that wires (452, 550, 552, 554) may be loosely positioned in channel (326) in the bunched-up, serpentine configuration as shown in FIG. 11A.

While the foregoing example provides the formation of wires (452, 550, 552, 554) to the bunched-up, serpentine configuration to provide slack that accommodates elongation imparted by bending of steerable portion (320), wires (452, 550, 552, 554) may be arranged in any other suitable configuration to accommodate elongation imparted by bending of steerable portion (320). For instance, in some variations, one or more of wires (452, 550, 552, 554) are routed circumferentially about steerable portion (320).

The relationship between wires (452, 550, 552, 554) and flex circuits (400, 500) may also accommodate freedom of movement of wires (452, 550, 552, 554) within channel (326) during bending of steerable portion (320) while also maintaining integrity in the connections between wires (452, 550, 552, 554) and connection pads (441, 440, 442, 444). For instance, in the region (R₁) of distal flex circuit (500) associated with coil portion (532), wires (550, 552, 554) may be tacked down to substrate (502) via an adhesive or in any other suitable fashion, thereby fixedly securing the portions of wires (550, 552, 554) in region (R₁) to substrate (502). Wires (550, 552, 554) further extend along tail portion (506) of flexible substrate (502) along region (R₂). In this region (R₂), wires (550, 552, 554) are not tacked down or otherwise secured to tail portion (506) of flexible substrate (502). Tail portion (506) is disposed in the distal region of channel (326). Within the distal region of channel (326), tail portion (506) is radially exterior to wires (550, 552, 554) yet still contained in flexible outer layer (324). Tail portion (506) may provide further strain relief to wires (550, 552, 554) along region (R₂), which corresponds to the distal region of channel (326) in which tail portion (506) is disposed. Tail portion (506) may thus protect wires (550, 552, 554), particularly during bending of steerable portion (320), where wires (550, 552, 554) enter channel (326).

Similarly, in the region (R₁) of proximal flex circuit (400) associated with coil portion (432), wires (452) may be tacked down to substrate (402) via an adhesive or in any other suitable fashion, thereby fixedly securing the portions of wires (452) in region (R₁) to substrate (402). Wires (452) further extend along tail portion (406) of flexible substrate (402) along region (R₂). In this region (R₂), wires (452) are not tacked down or otherwise secured to tail portion (406) of flexible substrate (402). Tail portion (406) is disposed in the proximal region of channel (326). Within the proximal region of channel (326), tail portion (406) is radially exterior to wires (452) yet still contained in flexible outer layer (324). Tail portion (406) may provide further strain relief to wires (452) along region (R₂), which corresponds to the proximal region of channel (326) in which tail portion (406) is disposed. Tail portion (406) may thus protect wires (452), particularly during bending of steerable portion (320), where wires (452) enter channel (326).

In some variations, flex circuits (400, 500) are integrated together into the same, single flex circuit. In some such variations, flex circuits (400, 500) share the same flexible substrate, with the shared flexible substrate including a strip that extends along channel (326). This strip may further include traces that effectively replace wires (452, 550, 552, 554). This strip may further bend with steerable portion (320) while the traces in the strip maintain electrical continuity with the electrical components of flex circuits (400, 500) described above. This strip may further extend proximally along the full length of proximal portion (310), such that the traces in the strip couple directly with corresponding connection points in handle assembly (110). As yet another variation, flex circuits (400, 500) may still be separate circuits that do not share a common flexible substrate, yet wires (550, 552, 554) may be replaced with traces formed on an elongate strip extension of substrate (502); and wires (452) may be replaced with traces formed on an elongate strip extension of substrate (402). As noted above, such elongate strip extensions of substrates (402, 502) may further extend proximally along the full length of proximal portion (310), such that the traces in the strip extensions couple directly with corresponding connection points in handle assembly (110). As yet another variation, electrical connections may be integrated into or through braided structure (322).

### V. Examples of Methods of Manufacturing Alternative Sheath Shaft Assembly with Integral Circuits

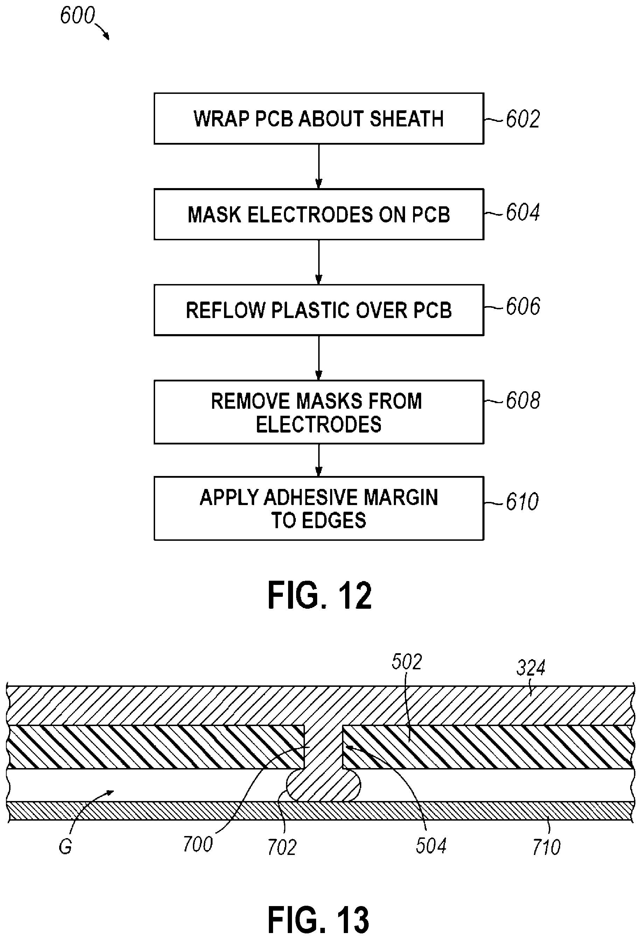

FIG. 12 shows an example of a method (600) of manufacturing shaft assembly (300). This example refers specifically to the method of assembling distal flex circuit (500) to distal portion (330) of shaft assembly (300). In this example, as shown in block (602), distal flex circuit (500) is wrapped about distal portion (330). Once distal flex circuit (500) has been wrapped about distal portion (330), electrode pads (520, 522) may be masked, as shown in block (604). By way of example only, polyimide material may be used to mask electrode pads (520, 522). Alternatively, any other suitable masking material(s) may be used.

Once electrode pads (520, 522) have been masked, a plastic material may be reflowed over distal flex circuit (500), as shown in block (606), thereby coating distal flex circuit (500) in a plastic material. Any suitable plastic reflow techniques may be used, including those using a combination of heat and pressure. Alternatively, distal flex circuit (500) may be coated in plastic material in any other suitable fashion. In some versions, the same plastic material that is used to coat distal flex circuit (500) is also used to form flexible outer layer (324) along steerable portion (320). By way of example only, the plastic material may include polyether block amide, thermoplastic polyurethane, or any other suitable plastic material. In the present example, the use of a plastic material to coat at least a portion of the exterior of shaft assembly (300) and flex circuits (400, 500) may provide a secure fit between flex circuits (400, 500) and shaft assembly (300) without substantially increasing the outer diameter of shaft assembly (300) in the regions where flex circuits (400, 500) are disposed.

Once distal flex circuit (500) has been sufficiently coated in plastic material, the masks are removed from electrode pads (520, 522), as shown in block (608). Electrode pads (520, 522) are thus exposed relative to the plastic material, such that electrode pads (520, 522) may achieve electrical continuity with blood, tissue, etc. As an optional finishing step, an adhesive or other material may be applied to the edges of the plastic material surrounding electrode pads (520, 522), as shown in block (610). By way of example only, this adhesive may include polyurethane a UV cure epoxy, or any other suitable material(s). The use of an adhesive margin may effectively smooth out the edges of the reflowed plastic material where electrode pads (520, 522) were masked, to the extent that such smoothing is warranted. In lieu of applying adhesive, the plastic material may be reflowed a second time to smooth out any otherwise rough edges.

FIG. 13 depicts a role that may be played by openings (504) in substrate (502) as part of the manufacture of shaft assembly (300) in accordance with method (600) of FIG. 12. For instance, the configuration shown in FIG. 13 may be achieved during the plastic reflow process described above with reference to block (606); or during the heat and compression process described above with reference to block (656). As shown, when the plastic material forming flexible outer layer (324) is in a flowable state, some of the flowing plastic material passes through opening (504). The plastic material eventually reaches a grounding structure (710) on the radially inner side of substrate (502). In some versions, grounding structure (710) is provided by distal portion (330) of shaft assembly (300). In some other versions, grounding structure (710) is provided by some other component that is radially interposed between distal portion (330) of shaft assembly (300) and substrate (502). In either scenario, the flowing plastic material may pass through a gap (G) defined between grounding structure (710) and substrate (502). The depiction of gap (G) in FIG. 13 is exaggerated for illustrative purposes only. As the flowing plastic material flows into gap (G), some of the flowing plastic material spreads within gap (G). The plastic material eventually hardens. At this point, as shown in FIG. 13, the portion of the plastic material disposed in opening (504) is in the form of a shaft (700), while the portion of the plastic material disposed in gap (G) is in the form of a head (702). The hardened plastic material thus forms a rivet shape. In some versions, the plastic material forming flexible outer layer (324) is compatible with the material forming grounding structure (710), such that these materials have similar melt points. Head (702) may thus fuse with grounding structure (710).

The rivet shape shown in FIG. 13 secures distal flex circuit (500) relative to flexible outer layer (324). While only one opening (504) is shown in FIG. 13, it should be understood that similar rivets may be formed in connection with the rest of openings (504) of substrate (502). Similarly, the same kinds of rivets may be formed in connection with openings (404) of substrate (402), such that the rivets may secure proximal flex circuit (400) relative to flexible outer layer (324). In some versions, flexible outer layer (324) extends along at least part of the length of distal portion (320), the full length of steerable portion (320), and at least part of the length of proximal portion (310). Flexible outer layer (324) may thus secure both flex circuits (400, 500) relative to the rest of shaft assembly (300). Alternatively, any other suitable kinds of structures or techniques may be used to secure either or both of flex circuits (400, 500) relative to the rest of shaft assembly (300).

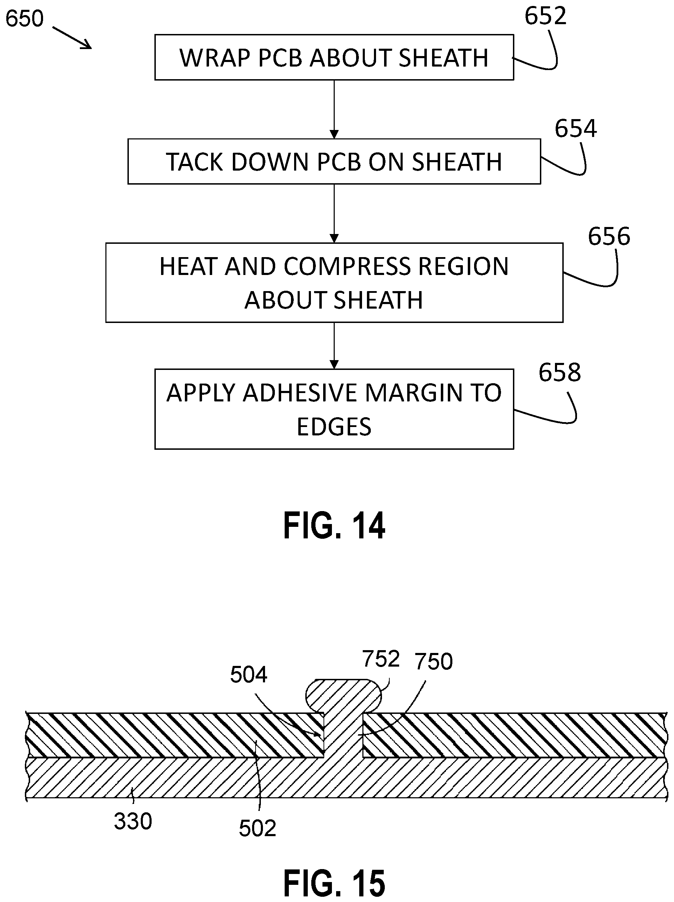

FIG. 14 shows an example of another method (650) of manufacturing shaft assembly (300). This example again refers specifically to the method of assembling distal flex circuit (500) to distal portion (330) of shaft assembly (300). In this example, as shown in block (652), distal flex circuit (500) is wrapped about distal portion (330). Once distal flex circuit (500) has been wrapped about distal portion (330), distal flex circuit (500) is tacked down to distal portion (330), as shown in block (654). In some versions, distal flex circuit (500) is tacked down to distal portion (330) via an adhesive. Alternatively, distal flex circuit (500) may be tacked down to distal portion (330) in any other suitable fashion. In still other versions, distal flex circuit (500) is not tacked down to distal portion (330), such that block (654) may be omitted in some versions.

Once distal flex circuit (500) has been wrapped about distal portion (330), and distal flex circuit (500) has been tacked down to distal portion (330) (if warranted), heat and compression are applied about the region of distal portion (330) where distal flex circuit (500) is wrapped, as shown in block (656). The combination of heat and compression may soften the material of distal portion (330) underlying distal flex circuit (500), such that distal flex circuit (500) becomes embedded in this material of distal portion (330). Various suitable components and techniques that may be used to apply heat and compression will be apparent to those skilled in the art in view of the teachings herein.

After distal flex circuit (500) has become embedded in this material of distal portion (330) due to the heat and compression described above, an adhesive margin may be applied around the perimeter of distal flex circuit (500), as shown in block (658). By way of example only, this adhesive may include polyurethane a UV cure epoxy, or any other suitable material(s). The use of an adhesive margin may effectively smooth out the edges as described above in the context of method (600). In some variations, the act of applying an adhesive margin is omitted.

FIG. 15 depicts a role that may be played by openings (504) in substrate (502) as part of the manufacture of shaft assembly (300) in accordance with method (650) of FIG. 14. For instance, the configuration shown in FIG. 15 may be achieved during the heat and compression process described above with reference to block (656). As shown, when the plastic material forming distal portion (330) softens, and as substrate (502) is compressed against the softened plastic material forming distal portion (330), some of the plastic material forming distal portion (330) passes through opening (504). After passing through opening (504), some of the softened plastic material spreads outwardly. The plastic material eventually hardens. At this point, as shown in FIG. 15, the portion of the plastic material disposed in opening (504) is in the form of a shaft (750), while the portion of the plastic material exposed relative to substrate (502) is in the form of a head (752). The hardened plastic material thus forms a rivet shape.

The rivet shape shown in FIG. 15 secures distal flex circuit (500) relative to flexible outer layer (324). While only one opening (504) is shown in FIG. 15, it should be understood that similar rivets may be formed in connection with the rest of openings (504) of substrate (502). Similarly, the same kinds of rivets may be formed in connection with openings (404) of substrate (402), such that the rivets may secure proximal flex circuit (400) relative to flexible outer layer (324). Alternatively, any other suitable kinds of structures or techniques may be used to secure either or both of flex circuits (400, 500) relative to the rest of shaft assembly (300).

The foregoing examples of methods (600, 650) are merely illustrative. Any other suitable methods may be used to assemble distal flex circuit (500) to distal portion (330) of shaft assembly (300). In some variations, the masking steps (blocks (604, 608)) are omitted; and the reflowed plastic material initially covers electrode pads (520, 522). After the reflow is complete and the plastic material is cured, the portion of the plastic covering electrode pads (520, 520) may be removed using a mechanical feature (e.g., a blade), using energy (e.g., a laser), or using any other suitable features or techniques. By way of further example only, distal flex circuit (500) may be adhered to distal portion (330) using an adhesive interposed between distal flex circuit and distal portion (330). Another configuration may include an adhesive margin only, with no additional plastic reflow layer and no adhesive between the distal flex circuit (500) and distal portion (330). The masking may be omitted in such versions. By way of further example only, distal flex circuit (500) may be printed directly on distal portion (330). By way of further example only, a sticker may be applied over distal flex circuit (500) to adhesively secure distal flex circuit (500) to distal portion (330). By way of further example only, distal flex circuit (500) may be ultrasonically welded to distal portion (330). Some variations may also provide distal flex circuit (500) in a preformed ring shape before distal flex circuit (500) is placed about distal portion (330), rather than having distal flex circuit (500) wrapped about distal portion (330) from a flat state as described above.

It should also be understood that proximal flex circuit (400) may be assembled to proximal portion (310) of shaft assembly (300) using a plastic reflow process similar to the plastic reflow process described above with reference to block (606); or using any of the alternative techniques referred to above. In versions where either flex circuit (400, 500) lacks electrode pads like electrode pads (520, 522), masking and unmasking steps (blocks (604, 608)) and adhesive margin application step (block (610)) may be omitted.

### VI. Example of Method of Using Alternative Sheath Shaft Assembly with Integral Circuits

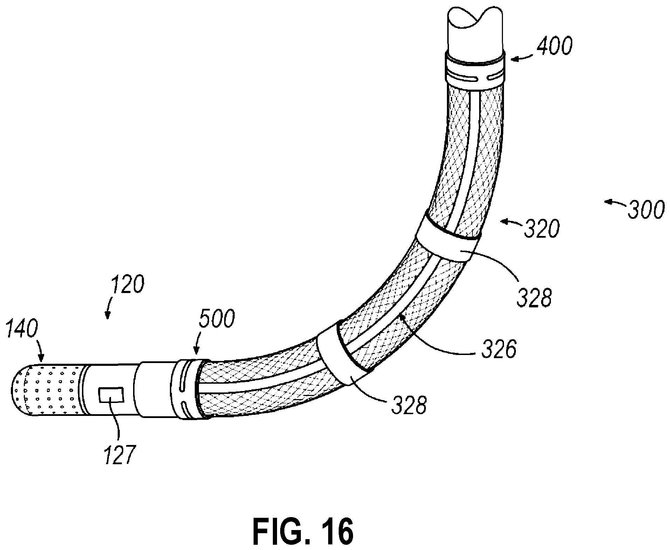

FIG. 16 shows an example of catheter (120) being used with a version of guiding sheath (200) that includes shaft assembly (300). As shown, rotary knob (214) has been actuated to laterally deflect distal portion (330), such that steerable portion (320) is in a bent state. Catheter (120) has been advanced distally through shaft assembly (300) such that end effector (140) projects distally relative to distal portion (330) or outside of shaft assembly (300). In this state of operation, the first type (e.g., electromagnetic-based) type of navigation sensor provided by proximal flex circuit (400) will generate signals indicating the real-time position of proximal flex circuit (400). In versions where proximal flex circuit (400) further includes the second type (e.g., impedance-based) type of navigation sensor, the position data provided by this second type of navigation sensor may supplement the position data from the first type of navigation sensor indicating the real-time position of proximal flex circuit (400). Similarly, in the present example, the first type (e.g., electromagnetic-based) and second type (e.g., impedance based) of navigation sensors provided by distal flex circuit (500) will generate signals indicating the real-time position of distal flex circuit (500). That is, the first type of navigation sensor (e.g., formed by coil portions (510, 512, 530, 532, 534) and second type of navigation sensor (e.g., formed by electrode pads (520, 522)), of which both types are collectively provided by distal flex circuit (500), will generate signals indicating the real-time position of distal flex circuit (500). Simultaneously, navigation sensor assembly (127) will generate signals indicating the real-time position of end effector (140). All of these signals may be processed by the processor of console (12) of guidance and drive system (10) to update the patient anatomy views in display (18) in real time, to depict a representation of shaft assembly (300) and end effector (140) in the regions of patient anatomy around end effector (140) and shaft assembly (300) as end effector (140) and shaft assembly (300) move within the patient (PA).

In addition to enabling depiction of the discrete real-time locations of flex circuits (400, 500) on display (18), the navigation sensing capabilities of flex circuits (400, 500) may enable depiction of the real-time shape of steerable portion (320) on display (18). The processor of console (12) may use interpolation techniques to determine the real-time shape of steerable portion (320) based on the real-time position data from flex circuits (400, 500). Providing the real-time shape of steerable portion (320) on display (18) may further enhance the understanding by the physician (PH) of how shaft assembly (300) is positioned and oriented within the anatomy of the patient (PA), which may in turn enhance the predictability of where end effector (140) will be positioned within the patient (PA) when end effector (140) is advanced distally from shaft assembly (300) as shown in FIG. 16. Once end effector (140) has been advanced distally from shaft assembly (300), end effector (140) may be utilized to perform an EP mapping procedure, perform an ablation procedure, and/or perform any other suitable kind(s) of procedure(s). While catheter (120) is shown and described herein as being usable with shaft assembly (300), it should be understood that shaft assembly (300) may be used with any other suitable kind(s) of catheter(s) and/or other kind(s) of instrumentation.

While the examples described herein provide incorporation of flex circuits (400, 500) in a shaft assembly (300) of a guiding sheath (200) with a steerable portion (320), flex circuits (400, 500) may be incorporated into other kinds of instruments. For instance, flex circuits (400, 500) may be incorporated into a shaft assembly of a guiding sheath that lacks a steerable portion like steerable portion (320). As another example, flex circuits (400, 500) may be incorporated into a shaft of a dilator instrument, catheter, or other elongated member.

### VIII. Miscellaneous

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

It should be understood that any of the examples described herein may include various other features in addition to or in lieu of those described above.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein.

Having shown and described various versions of the present invention, further adaptations of the systems described herein may be accomplished by appropriate modifications by one skilled in the art, provided that they fall under the scope of the claims. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention is defined by the following claims and is understood not to be limited to the details of structure and operation shown and described in the specification and drawings.

## Claims

1. A guiding sheath (200) for guiding a catheter (120) to a target location within a patient, the guiding sheath comprising:
(a) a body (210); and
(b) a hollow shaft assembly (300) extending distally from the body (210), the shaft assembly (300) including:
(i) a proximal portion (310) defining a longitudinal axis,
(ii) a steerable portion (320) distal to the proximal portion (310),
(iii) a distal portion (330) distal to the steerable portion (320), the steerable portion (320) being operable to drive the distal portion (330) laterally away from and toward the longitudinal axis,
(iv) at least one flex circuit assembly (400, 500), the at least one flex circuit assembly (400, 500) including:
(A) a first navigation sensor (520, 522) configured to measure impedance signals that indicate a real-time position of the steerable portion (320), and
(B) a second navigation sensor (510, 512) configured to measure magnetic signals that indicate a real-time position of the steerable portion (320).

2. The guiding sheath (200) of claim 1, the first navigation sensor (520, 522) comprising a flexible substrate and at least one electrode disposed on the flexible substrate to measure impedance and generate a real-time position of the at least one electrode.

3. The guiding sheath (200) of claim 1 or claim 2, the second navigation sensor (510, 512) comprising a flexible substrate and at least one coil portion disposed on the flexible substrate to measure magnetic signals and generate a real-time position of the at least one coil portion,
preferably wherein the at least one coil portion comprises a plurality of coil portions disposed on the flexible substrate, the plurality of coil portions being coupled together to collectively form the second navigation sensor (510, 512),
further preferably wherein the plurality of coil portions comprises a first coil portion (510) and a second coil portion (512), the first coil portion and the second coil portion being positioned at diametrically opposing locations about a circumference of the shaft assembly (300).

4. The guiding sheath (200) of any of claims 1 to 3, the at least one flex circuit assembly (400) being positioned at a distal end of the proximal portion (310) of the shaft assembly (300).

5. The guiding sheath (200) of any one of claims 1 to 3, the at least one flex circuit assembly (500) being positioned at a proximal end of the steerable portion (320).

6. The guiding sheath (200) of any preceding claim, further comprising a second flex circuit assembly (500) including a flexible substrate and at least one coil portion (530, 532, 534) disposed on the flexible substrate, the at least one coil portion forming a navigation sensor.

7. The guiding sheath (200) of any preceding claim, the at least one flex circuit assembly (400, 500) including a first flex circuit assembly (400) and a second flex circuit assembly (500) , each of the first and second flex circuit assembly including:
(A) a first navigation sensor (520, 522) configured to measure impedance signals that indicate a real-time position of the steerable portion (320), and
(B) a second navigation sensor (510, 512) configured to measure magnetic signals that indicate a real-time position of the steerable portion (320).

8. The guiding sheath (200) of claim 7, the second flex circuit assembly (500) being angularly offset from the first flex circuit assembly about a circumference of the shaft assembly (300), preferably wherein the second flex circuit assembly being angularly offset from the first flex circuit assembly by about 90 degrees.

9. The guiding sheath (200) of any preceding claim, the first navigation sensor (520, 522) comprising a plurality of exposed electrodes, the second navigation sensor (510, 512) comprising a plurality of coils.

10. The guiding sheath (200) of any preceding claim, each of the first and second navigation sensors (520, 522, 510, 512) further comprising a plurality of connection pads (540, 542, 554) disposed on the at least one flexible circuit assembly, the apparatus further comprising a plurality of wires secured to the connection pads.

11. The guiding sheath (200) of claim 10, the shaft assembly (300) further including a channel (326), the wires being disposed in the channel, the channel extending along the steerable portion (320).

12. The guiding sheath (200) of claim 11, the wires being configured to transition between a first configuration in the channel and a second configuration in the channel, the wires in the first configuration defining a service loop providing freedom of movement of the wires in the channel, the wires in the second configuration being elongated in response to bending of the steerable portion (320), optionally wherein the service loop comprises a serpentine shape or the wires are resiliently biased to assume the first configuration.

13. The guiding sheath (200) of any preceding claim, the shaft assembly (300) further comprising one or more ring electrodes (328).

14. The guiding sheath (200) of claim 1,
wherein the body is a handle assembly;
wherein the steerable portion (320) is operable to bend in response to actuation of the actuator to thereby drive the distal portion (330) laterally away from and toward the longitudinal axis, the distal portion (330) having an open distal end,
wherein the at least one flex circuit assembly (500) is positioned at a distal end of the steerable portion (320);
wherein the shaft assembly further comprises a second flex circuit assembly (400) positioned at a proximal end of the steerable portion (320), the second flex circuit assembly including:
(A) a first navigation sensor (520, 522) configured to measure impedance signals that indicate a real-time position of the steerable portion (320), and
(B) a second navigation sensor (510, 512) configured to measure magnetic signals that indicate a real-time position of the steerable portion (320).

15. A method of manufacturing a shaft assembly of a guiding sheath according to any preceding claim, the method comprising:
(a) positioning a first flex circuit assembly (400) at a distal end of a proximal portion (310) of a shaft assembly (300), the first flex circuit assembly including a flexible substrate and one or more electrodes configured to sense impedance as a first navigation sensor (520, 522) and one or more coil portions configured to sense magnetic fields as a second navigation sensor (510, 512);
(b) positioning a second flex circuit assembly (500) at a proximal end of a distal portion (330) of the shaft assembly (300), the shaft assembly (300) further including a steerable portion (320) extending between the proximal portion (310) and the distal portion (330), the second flex circuit assembly including one or more electrodes configured to sense impedance as a first navigation sensor (520, 522) and one or more coil portions configured to sense magnetic fields; and
(c) flowing a plastic material through one or more openings formed through the first and second flex circuit assemblies (400, 500), thereby securing the first flex circuit assembly to the proximal portion of the shaft assembly (300), and thereby securing the second flex circuit assembly to the distal portion (330) of the shaft assembly (300).

## Patentansprüche

1. Führungshülse (200) zum Führen eines Katheters (120) zu einer Zielstelle innerhalb eines Patienten, die Führungshülse umfassend:
(a) einen Körper (210); und
(b) eine hohle Schaftanordnung (300), die sich von dem Körper (210) distal erstreckt, wobei die Schaftanordnung (300) einschließt:
(i) einen proximalen Abschnitt (310), der eine Längsachse definiert,
(ii) einen lenkbaren Abschnitt (320), der zu dem proximalen Abschnitt (310) distal ist,
(iii) einen distalen Abschnitt (330), der zu dem lenkbaren Abschnitt (320) distal ist, wobei der lenkbare Abschnitt (320) betriebsfähig ist, um den distalen Abschnitt (330) seitlich von der Längsachse weg und zu dieser hin zu treiben,
(iv) mindestens eine Flexschaltungsanordnung (400, 500), wobei die mindestens eine Flexschaltungsanordnung (400, 500) einschließt:
(A) einen ersten Navigationssensor (520, 522), der konfiguriert ist, um Impedanzsignale zu messen, die eine Echtzeitposition des lenkbaren Abschnitts (320) anzeigen, und
(B) einen zweiten Navigationssensor (510, 512), der konfiguriert ist, um magnetische Signale zu messen, die eine Echtzeitposition des lenkbaren Abschnitts (320) anzeigen.

2. Führungshülse (200) nach Anspruch 1, der erste Navigationssensor (520, 522) umfassend ein flexibles Substrat und mindestens eine Elektrode, die auf dem flexiblen Substrat angeordnet ist, um eine Impedanz zu messen und eine Echtzeitposition der mindestens einen Elektrode zu erzeugen.

3. Führungshülse (200) nach Anspruch 1 oder 2, der zweite Navigationssensor (510, 512) umfassend ein flexibles Substrat und mindestens einen Spulenabschnitt, der auf dem flexiblen Substrat angeordnet ist, um magnetische Signale zu messen und eine Echtzeitposition des mindestens einen Spulenabschnitts zu erzeugen,
vorzugsweise wobei der mindestens eine Spulenabschnitt eine Vielzahl von Spulenabschnitten umfasst, die auf dem flexiblen Substrat angeordnet ist, wobei die Vielzahl von Spulenabschnitten miteinander gekoppelt ist, um den zweiten Navigationssensor (510, 512) gemeinsam auszubilden,
ferner bevorzugt, wobei die Vielzahl von Spulenabschnitten einen ersten Spulenabschnitt (510) und einen zweiten Spulenabschnitt (512) umfasst, wobei der erste Spulenabschnitt und der zweite Spulenabschnitt an diametral gegenüberliegenden Stellen um einen Umfang der Schaftanordnung (300) herum positioniert sind.

4. Führungshülse (200) nach einem der Ansprüche 1 bis 3, wobei die mindestens eine Flexschaltungsanordnung (400) an einem distalen Ende des proximalen Abschnitts (310) der Schaftanordnung (300) positioniert ist.

5. Führungshülse (200) nach einem der Ansprüche 1 bis 3, wobei die mindestens eine Flexschaltungsanordnung (500) an einem proximalen Ende des lenkbaren Abschnitts (320) positioniert ist.

6. Führungshülse (200) nach einem der vorstehenden Ansprüche, ferner umfassend eine zweite Flexschaltungsanordnung (500), die ein flexibles Substrat und mindestens einen Spulenabschnitt (530, 532, 534) einschließt, der auf dem flexiblen Substrat angeordnet ist, wobei der mindestens eine Spulenabschnitt einen Navigationssensor ausbildet.

7. Führungshülse (200) nach einem der vorstehenden Ansprüche, wobei die mindestens eine Flexschaltungsanordnung (400, 500) eine erste Flexschaltungsanordnung (400) und eine zweite Flexschaltungsanordnung (500) einschließt, wobei jede der ersten und der zweiten Flexschaltungsanordnung einschließt:
(A) einen ersten Navigationssensor (520, 522), der konfiguriert ist, um Impedanzsignale zu messen, die eine Echtzeitposition des lenkbaren Abschnitts (320) anzeigen, und
(B) einen zweiten Navigationssensor (510, 512), der konfiguriert ist, um magnetische Signale zu messen, die eine Echtzeitposition des lenkbaren Abschnitts (320) anzeigen.

8. Führungshülse (200) nach Anspruch 7, wobei die zweite Flexschaltungsanordnung (500) von der ersten Flexschaltungsanordnung um einen Umfang der Schaftanordnung (300) herum winkelversetzt ist, vorzugsweise wobei die zweite Flexschaltungsanordnung von der ersten Flexschaltungsanordnung um etwa 90 Grad winkelversetzt ist.

9. Führungshülse (200) nach einem der vorstehenden Ansprüche, der erste Navigationssensor (520, 522) umfassend eine Vielzahl von freiliegenden Elektroden, der zweite Navigationssensor (510, 512) umfassend eine Vielzahl von Spulen.

10. Führungshülse (200) nach einem der vorstehenden Ansprüche, jeder des ersten und des zweiten Navigationssensors (520, 522, 510, 512) ferner umfassend eine Vielzahl von Anschlussflächen (540, 542, 554), die auf der mindestens einen Flexschaltungsanordnung angeordnet sind, die Einrichtung ferner umfassend eine Vielzahl von Drähten, die an den Anschlussflächen befestigt ist.

11. Führungshülse (200) nach Anspruch 10, wobei die Schaftanordnung (300) ferner einen Kanal (326) einschließt, wobei die Drähte in dem Kanal angeordnet sind, der Kanal sich entlang des lenkbaren Abschnitts (320) erstreckt.

12. Führungshülse (200) nach Anspruch 11, wobei die Drähte konfiguriert sind, um zwischen einer ersten Konfiguration in dem Kanal und einer zweiten Konfiguration in dem Kanal zu wechseln, wobei die Drähte in der ersten Konfiguration eine Serviceschleife definieren, die Bewegungsfreiheit der Drähte in dem Kanal bereitstellt, wobei die Drähte in der zweiten Konfiguration als Reaktion auf ein Biegen des lenkbaren Abschnitts (320) verlängert werden, optional wobei die Serviceschleife eine Serpentinenform umfasst oder die Drähte elastisch vorgespannt sind, um die erste Konfiguration einzunehmen.

13. Führungshülse (200) nach einem der vorstehenden Ansprüche, die Schaftanordnung (300) ferner umfassend eine oder mehrere Ringelektroden (328).

14. Führungshülse (200) nach Anspruch 1,
wobei der Körper eine Griffanordnung ist;
wobei der lenkbare Abschnitt (320) als Reaktion auf eine Betätigung des Aktuators betriebsfähig ist, um gebogen zu werden, wobei dadurch der distale Abschnitt (330) seitlich von der Längsachse weg und zu dieser hin getrieben wird, wobei der distale Abschnitt (330) ein offenes distales Ende aufweist,
wobei die mindestens eine Flexschaltungsanordnung (500) an einem distalen Ende des lenkbaren Abschnitts (320) positioniert ist;
wobei die Schaftanordnung ferner eine zweite Flexschaltungsanordnung (400) umfasst, die an einem proximalen Ende des lenkbaren Abschnitts (320) positioniert ist, wobei die zweite Flexschaltungsanordnung einschließt:
(A) einen ersten Navigationssensor (520, 522), der konfiguriert ist, um Impedanzsignale zu messen, die eine Echtzeitposition des lenkbaren Abschnitts (320) anzeigen, und
(B) einen zweiten Navigationssensor (510, 512), der konfiguriert ist, um magnetische Signale zu messen, die eine Echtzeitposition des lenkbaren Abschnitts (320) anzeigen.

15. Verfahren zum Herstellen einer Schaftanordnung einer Führungshülse nach einem der vorstehenden Ansprüche, das Verfahren umfassend:
(a) Positionieren einer ersten Flexschaltungsanordnung (400) an einem distalen Ende eines proximalen Abschnitts (310) einer Schaftanordnung (300), wobei die erste Flexschaltungsanordnung ein flexibles Substrat und eine oder mehrere Elektroden, die konfiguriert sind, um die Impedanz als einen ersten Navigationssensor (520, 522) zu erfassen, und einen oder mehrere Spulenabschnitte einschließt, die konfiguriert sind, um magnetische Felder als einen zweiten Navigationssensor (510, 512) zu erfassen;
(b) Positionieren einer zweiten Flexschaltungsanordnung (500) an einem proximalen Ende eines distalen Abschnitts (330) der Schaftanordnung (300), wobei die Schaftanordnung (300) ferner einen lenkbaren Abschnitt (320) einschließt, der sich zwischen dem proximalen Abschnitt (310) und dem distalen Abschnitt (330) erstreckt, die zweite Flexschaltungsanordnung eine oder mehrere Elektroden, die konfiguriert sind, um die Impedanz als einen erster Navigationssensor (520, 522) zu erfassen, und einen oder mehrere Spulenabschnitte einschließt, die konfiguriert sind, um magnetische Felder zu erfassen; und
(c) Fließen eines Kunststoffmaterials durch eine oder mehrere Öffnungen, die durch die erste und die zweite Flexschaltungsanordnung (400, 500) ausgebildet sind, wobei dadurch die erste Flexschaltungsanordnung an dem proximalen Abschnitt der Schaftanordnung (300) befestigt wird und wobei dadurch die zweite Flexschaltungsanordnung an dem distalen Abschnitt (330) der Schaftanordnung (300) befestigt wird.

## Revendications

1. Gaine de guidage (200) permettant de guider un cathéter (120) vers un emplacement cible à l'intérieur d'un patient, la gaine de guidage comprenant :
(a) un corps (210) ; et
(b) un ensemble arbre creux (300) s'étendant de manière distale à partir du corps (210), l'ensemble arbre (300) comportant :
(i) une partie proximale (310) définissant un axe longitudinal,
(ii) une partie orientable (320) distale par rapport à la partie proximale (310),
(iii) une partie distale (330) distale par rapport à la partie orientable (320), la partie orientable (320) pouvant entraîner la partie distale (330) latéralement loin de l'axe longitudinal et vers celui-ci,
(iv) au moins un ensemble circuit souple (400, 500), l'au moins un ensemble circuit souple (400, 500) comportant :
(A) un premier capteur de navigation (520, 522) configuré pour mesurer des signaux d'impédance qui indiquent une position en temps réel de la partie orientable (320), et
(B) un second capteur de navigation (510, 512) configuré pour mesurer des signaux magnétiques qui indiquent une position en temps réel de la partie orientable (320).

2. Gaine de guidage (200) selon la revendication 1, le premier capteur de navigation (520, 522) comprenant un substrat souple et au moins une électrode disposée sur le substrat souple pour mesurer l'impédance et générer une position en temps réel de l'au moins une électrode.

3. Gaine de guidage (200) selon la revendication 1 ou la revendication 2, le second capteur de navigation (510, 512) comprenant un substrat souple et au moins une partie de bobine disposée sur le substrat souple pour mesurer des signaux magnétiques et générer une position en temps réel de l'au moins une partie de bobine,
de préférence, dans lequel l'au moins une partie de bobine comprend une pluralité de parties de bobine disposées sur le substrat souple, la pluralité de parties de bobine étant couplées ensemble pour former collectivement le second capteur de navigation (510, 512),
de préférence, dans laquelle la pluralité de portions de bobines comprend une première partie de bobine (510) et une seconde partie de bobine (512), la première partie de bobine et la seconde partie de bobine étant positionnées à des emplacements diamétralement opposés sur la circonférence de l'ensemble arbre (300).

4. Gaine de guidage (200) selon l'une quelconque des revendications 1 à 3, l'au moins un ensemble circuit souple (400) étant positionné à une extrémité distale de la partie proximale (310) de l'ensemble arbre (300).

5. Gaine de guidage (200) selon l'une quelconque des revendications 1 à 3, l'au moins un circuit souple (500) étant positionné à une extrémité proximale de la partie orientable (320).

6. Gaine de guidage (200) selon l'une quelconque revendication précédente, comprenant en outre un second ensemble circuit souple (500) comportant un substrat souple et au moins une partie de bobine (530, 532, 534) disposée sur le substrat souple, l'au moins une partie de bobine formant un capteur de navigation.

7. Gaine de guidage (200) selon l'une quelconque revendication précédente, l'au moins un ensemble circuit souple (400, 500) comportant un premier ensemble circuit souple (400) et un second ensemble circuit souple (500), chacun des premier et second ensembles circuit souple comportant :
(A) un premier capteur de navigation (520, 522) configuré pour mesurer des signaux d'impédance qui indiquent une position en temps réel de la partie orientable (320), et
(B) un second capteur de navigation (510, 512) configuré pour mesurer des signaux magnétiques qui indiquent une position en temps réel de la partie orientable (320).

8. Gaine de guidage (200) selon la revendication 7, le second circuit souple (500) étant décalé de manière angulaire à partir du premier circuit souple sur une circonférence de l'ensemble arbre (300), de préférence dans laquelle le second circuit souple est décalé de manière angulaire à partir du premier circuit souple d'environ 90 degrés.

9. Gaine de guidage (200) selon l'une quelconque revendication précédente, le premier capteur de navigation (520, 522) comprenant une pluralité d'électrodes exposées, le second capteur de navigation (510, 512) comprenant une pluralité de bobines.

10. Gaine de guidage (200) selon l'une quelconque revendication précédente, chacun des premier et second capteurs de navigation (520, 522, 510, 512) comprenant en outre une pluralité de plots de connexion (540, 542, 554) disposés sur l'au moins un ensemble circuit souple, l'appareil comprenant en outre une pluralité de fils fixés aux plots de connexion.

11. Gaine de guidage (200) selon la revendication 10, l'ensemble arbre (300) comportant en outre un canal (326), les fils étant disposés dans le canal, le canal s'étendant le long de la partie orientable (320).

12. Gaine de guidage (200) selon la revendication 11, les fils étant configurés pour passer d'une première configuration dans le canal à une seconde configuration dans le canal, les fils dans la première configuration définissant une boucle de service assurant la liberté de déplacement des fils dans le canal, les fils dans la seconde configuration étant allongés en réponse à la flexion de la partie orientable (320), éventuellement dans laquelle la boucle de service comprend une forme serpentine ou les fils sont élastiquement sollicités pour adopter la première configuration.

13. Gaine de guidage (200) selon l'une quelconque revendication précédente, l'ensemble arbre (300) comprenant en outre une ou plusieurs électrodes annulaires (328).

14. Gaine de guidage (200) selon la revendication 1,
dans laquelle le corps est un ensemble poignée ;
dans laquelle la partie orientable (320) peut se plier en réponse à l'actionnement de l'actionneur pour ainsi entraîner la partie distale (330) latéralement loin de l'axe longitudinal et vers celui-ci, la partie distale (330) présentant une extrémité distale ouverte,
dans laquelle l'au moins un ensemble circuit souple (500) est positionné au niveau d'une extrémité distale de la partie orientable (320) ;
dans laquelle l'ensemble arbre comprend en outre un second ensemble circuit souple (400) positionné au niveau d'une extrémité proximale de la partie orientable (320), le second ensemble circuit souple comportant :
(A) un premier capteur de navigation (520, 522) configuré pour mesurer des signaux d'impédance qui indiquent une position en temps réel de la partie orientable (320), et
(B) un second capteur de navigation (510, 512) configuré pour mesurer des signaux magnétiques qui indiquent une position en temps réel de la partie orientable (320).

15. Procédé de fabrication d'un ensemble arbre d'une gaine de guidage selon l'une quelconque revendication précédente, le procédé comprenant :
(a) le positionnement d'un premier circuit souple (400) au niveau d'une extrémité distale d'une partie proximale (310) d'un ensemble arbre (300), le premier circuit souple comportant un substrat souple et une ou plusieurs électrodes configurées pour détecter l'impédance comme premier capteur de navigation (520, 522) et une ou plusieurs parties de bobine configurées pour détecter des champs magnétiques comme second capteur de navigation (510, 512) ;
(b) le positionnement d'un second circuit souple (500) au niveau d'une extrémité proximale d'une partie distale (330) de l'ensemble arbre (300), l'ensemble arbre (300) comportant en outre une partie orientable (320) s'étendant entre la partie proximale (310) et la partie distale (330), le second circuit souple comportant une ou plusieurs électrodes configurées pour détecter l'impédance comme premier capteur de navigation (520, 522) et une ou plusieurs parties de bobine configurées pour détecter des champs magnétiques ; et
(c) l'écoulement d'une matière plastique à travers une ou plusieurs ouvertures formées dans les premier et second ensembles circuit souple (400, 500), fixant ainsi le premier ensemble circuit souple à la partie proximale de l'ensemble arbre (300), et fixant ainsi le second ensemble circuit souple à la partie distale (330) de l'ensemble arbre (300).
